# FASCICULE DE BREVET EUROPEEN

(11) **EP 2 129 418 B1**
(45) Date de publication et mention de la délivrance du brevet: **13.04.2016**
(21) Numéro de dépôt: 08710086.3
(22) Date de dépôt: 19.02.2008
(51) Int. Cl.: A61M 5/315, A61M 5/24, A61M 5/34, A61M 5/31

(54) **DISPOSITIF D'EJECTION JETABLE D'UN PRODUIT LIQUIDE OU PATEUX**
EINWEGVORRICHTUNG ZUM AUSWERFEN EINER FLÜSSIGKEIT ODER EINES PASTÖSEN PRODUKTS
DISPOSABLE DEVICE FOR EJECTING A LIQUID OR PASTY PRODUCT

(30) Priorité: 07.03.2007 FR 0701649
(43) Date de publication de la demande: 09.12.2009
(73) Titulaire: Primequal S.A., 1268 Begnins (CH)
(72) Inventeur: WEILL, David, CH-1268 Begnins (CH); CHASSOT, Pierre-Yves, F-01710 Thoiry (FR)
(74) Mandataire: Bugnion Genève
(86) Numéro de dépôt international: PCT/IB2008/050593
(87) Numéro de publication internationale: WO 2008/107813

(56) Documents cités:
- EP-A- 0 276 474
- EP-A- 1 495 777
- WO-A-94/12228
- WO-A-2005/007224
- US-A- 5 570 821

## Description

L'invention tel que définie par les revendications annexées concerne un dispositif d'éjection d'un produit liquide ou pâteux, ainsi qu'un procédé de fabrication et de distribution du dispositif.

Il est connu de la demande de brevet FR 2 535 206 une seringue dentaire pour injection intra-ligamentaire. Cette seringue permet l'injection par une aiguille très fine et souple d'un produit dans les ligaments situés entre l'os de la mâchoire et la dent. Elle est principalement constituée d'un corps allongé sur lequel est monté un mécanisme commandant l'injection par le biais du déplacement d'un cylindre de poussée, d'un porte-conteneur dans lequel est logé un conteneur rempli de liquide à injecter et d'un embout comportant l'aiguille d'injection. Afin de résoudre des problèmes d'accès difficile aux zones où doivent être faites les injections, le corps de la seringue présente une tête d'injection qui fait un angle avec l'axe du corps de la seringue. L'aiguille, amovible, est mise en place sur le corps avant de pratiquer les injections puis retirée après. Le mécanisme commandant l'injection est composé principalement d'un levier articulé sur le corps de seringue et agissant sur un cylindre de poussée par le biais d'un cliquet articulé sur le levier et rappeler dans une position de contact avec les dents d'une crémaillère réalisées sur le cylindre de poussée. Le cylindre est guidé en translation dans un alésage réalisé dans le corps de seringue. Il présente en outre une rainure longitudinale coopérant avec une vis vissée radialement par rapport à l'alésage et débouchant dans celui-ci pour interdire la rotation du cylindre. Le mécanisme présente de plus un cliquet anti-retour interdisant le recul du cylindre de poussée lorsqu'on met fin à l'action sur le levier. Ce cliquet anti-retour est rappelé dans une position de contact avec les dents de la crémaillère et peut être éloigné de cette position par action sur un bouton pour annuler la pression d'injection et/ou pour changer le conteneur de produit à injecter sur lequel appuie le cylindre de poussée. Un tel dispositif présente des inconvénients. D'une part, sa réalisation est complexe et coûteuse. D'autre part, il présente de nombreuses pièces et des formes complexes, en particulier, des angles et des coins dans la matière. Ces angles et coins forment des zones très peu accessibles et, par conséquent, très difficiles à nettoyer et donc difficiles à stériliser.

Pour pallier aux inconvénients précédents, le document WO2005/007224 propose un dispositif d'éjection présentant une construction plus simple, aisément démontable et nettoyable. Ce dispositif comprend un corps, une partie destinée à contenir le produit et munie d'un orifice pour l'éjection du produit, un cylindre de poussée muni de dents, se déplaçant dans un alésage du corps et faisant varier le volume dé la partie destinée à contenir le produit et un mécanisme de déplacement du cylindre de poussée lié au corps, comprenant un levier articulé démontable, rappelé par un ressort de rappel, agissant sur les dents du cylindre de poussée par le biais d'un cliquet articulé sur le levier et rappelé dans une position de contact avec le cylindre de poussée par un ressort et un cliquet anti-retour rappelé dans une position de contact avec le cylindre de poussée. Malgré sa plus grande simplicité et la présence d'un levier démontable facilitant le nettoyage, ce dispositif n'atteint toujours pas un degré d'hygiène satisfaisant et nécessite toujours une phase de nettoyage délicate. De plus, la liaison démontable du levier avec le corps du dispositif est obtenue au détriment de l'efficacité du dispositif, car elle induit une moins bonne tenue du levier qui subit des efforts importants lors de la réalisation d'une éjection. Enfin, cette solution comme la précédente nécessite au moins un pré-assemblage partiel avant sa distribution, sinon son assemblage se révèlerait trop contraignant du fait du nombre d'éléments du dispositif.

Un autre exemple de l'art antérieure est décrit dans EP 0 276 474 A.

Ainsi, un objet général de l'invention est de proposer un dispositif d'éjection d'un produit liquide ou pâteux palliant aux inconvénients des solutions existantes.

Plus précisément, l'invention souhaite atteindre tout ou partie des objets particuliers suivants.

Un premier objet est de proposer un dispositif d'éjection d'un produit liquide ou pâteux offrant un degré d'hygiène maximal.

Un second objet est de proposer un dispositif d'éjection d'un produit liquide ou pâteux avec une grande efficacité d'éjection.

Un troisième objet est de proposer un procédé de fabrication et de distribution économique d'un dispositif d'éjection.

L'invention atteint les objets précédents sur la base d'un concept totalement différent de dispositif d'éjection jetable, grâce à un dispositif d'éjection encore simplifié et très peu coûteux.

Plus précisément, l'invention repose sur un dispositif d'éjection d'un produit liquide ou pâteux, comprenant un corps, ayant une partie destinée à contenir le produit à éjecter et munie d'un orifice pour l'éjection du produit, une crémaillère se déplaçant dans un alésage du corps et faisant varier le volume de la partie destinée à contenir le produit et un mécanisme de déplacement de la crémaillère, comprenant un levier articulé et un cliquet pour agir sur la crémaillère caractérisé en ce que le cliquet et le levier forment une structure monolithique en matériau plastique.

Pour cela, le cliquet peut être sensiblement perpendiculaire au levier et relié au levier en une zone de liaison présentant des découpes et/ou des zones de moindre épaisseur pour former une zone de moindre rigidité et déformable pour permettre un mouvement élastique relatif du cliquet et du levier. De plus, le cliquet peut présenter une flexibilité apte à une éjection en deux temps, un premier temps consistant en sa déformation sans éjection et un appui de plus en plus fort sur la crémaillère et un second temps consistant en l'avancée de la crémaillère et l'éjection.

Le levier peut comprendre un axe à son extrémité formant une seule structure monolithique avec le levier et le cliquet.

Enfin, le dispositif peut comprendre un moyen de verrouillage de la liaison entre le levier et le corps.

Pour cela, le corps du dispositif d'éjection peut comprendre un emplacement de forme correspondant à celle de l'axe pour loger l'axe, et au moins une lame élastique permettant de clipser l'axe du levier dans le logement du corps. Cette lame élastique peut être intégrée au corps, au niveau de la surface supérieure de l'alésage, de sorte de pouvoir se déformer au sein de cet alésage pour l'assemblage du levier sur le corps et de ne plus pouvoir se déformer et verrouiller le levier sur le corps lorsque la crémaillère est présente dans l'alésage.

La lame élastique peut présenter une surface supérieure arrondie épousant l'axe et assurant son bon positionnement dans l'emplacement lors de l'insertion de la crémaillère.

Selon une seconde solution avantageuse, le dispositif d'éjection peut comprendre une lame élastique intégrée au corps remplissant la fonction de cliquet anti-retour. L'extrémité de ce cliquet anti-retour peut se trouver positionnée en avant ou au même niveau que l'extrémité du cliquet lié au levier.

Selon une troisième solution avantageuse, le corps du dispositif d'éjection peut être composé de l'assemblage d'au moins un porte-conteneur et un corps arrière, le porte-conteneur représentant la partie avant du corps et le corps arrière sa partie arrière, les deux parties pouvant être reliées par au moins deux lames élastiques longitudinales d'une première partie positionnées dans des rails de la seconde partie. Les lames peuvent présenter une protubérance à leur extrémité pouvant se clipser dans des ouvertures de l'autre partie.

Selon une variante intéressante, la liaison entre les deux parties du corps est verrouillée ou quasi-verrouillée, entraînant le verrouillage de l'ensemble des composants assemblés pour une unique utilisation du dispositif qui est jetable. Selon une autre variante, la liaison entre les deux parties du corps est démontable pour permettre le désassemblage du dispositif pour son nettoyage.

Selon une réalisation préférée de l'invention, le dispositif d'éjection d'un produit liquide ou pâteux est composé de quatre parties principales assemblées, un levier incluant un cliquet, un porte-conteneur, un corps arrière et une crémaillère, au moins le levier, le porte-conteneur et le corps arrière pouvant être en matériau plastique injecté.

Selon une variante possible, au moins le porte-conteneur est en matériau plastique transparent.

Le corps du dispositif peut présenter une extrémité antérieure pour recevoir un porte-outil avec une aiguille, cette extrémité antérieure ayant une forme inclinée par rapport au corps et présentant des génératrices parallèles au corps pour permettre une insertion droite de l'aiguille.

Selon une dernière variante d'exécution, le dispositif d'éjection comprend une chemise dans le corps arrière.

L'invention porte aussi sur un procédé de fabrication d'un dispositif d'éjection comprenant au moins trois étapes d'injection de matériau plastique pour former trois parties distinctes du dispositif, un levier intégrant un cliquet, un porte-conteneur et un corps arrière. Il peut comprendre une quatrième étape d'injection en matériau plastique pour former une crémaillère.

En variante, le procédé de fabrication d'un dispositif d'éjection d'un produit liquide ou pâteux peut comprendre une étape de fabrication d'au moins quatre éléments distincts, un levier intégrant un cliquet, un porte-conteneur, un corps arrière et une crémaillère, et il peut comprendre un procédé d'assemblage de ces quatre éléments comprenant les étapes suivantes :
a. Insertion du levier sur le corps arrière,
b. Insertion de la crémaillère au sein d'un alésage du corps arrière et liaison du porte-conteneur à l'avant du corps arrière.

Enfin, l'invention porte sur un procédé de distribution d'un dispositif d'éjection d'un produit liquide ou pâteux caractérisé en ce qu'il comprend l'étape consistant à distribuer au moins quatre éléments distincts non assemblés, un levier intégrant un cliquet, un porte-conteneur, un corps arrière et une crémaillère.

Ces objets, caractéristiques et avantages, ainsi que d'autres de la présente invention seront exposés en détail dans la description suivante d'un mode d'exécution particulier fait à titre non-limitatif en relation avec les figures jointes parmi lesquelles :
Les figures 1 a à 1 d représentent schématiquement différentes étapes d'assemblage d'un dispositif d'éjection selon un mode de réalisation de l'invention ;
la figure 2 représente une vue schématique en coupe selon un plan vertical longitudinal passant approximativement au milieu du dispositif d'une partie du dispositif d'éjection selon le mode de réalisation de l'invention au niveau de la liaison du levier sur le corps ;
la figure 3 représente une vue en perspective de l'assemblage du dispositif correspondant à la figure 1b ;
la figure 4 représente une vue en perspective du dispositif assemblé ;
la figure 5 représente une vue partielle en perspective de l'assemblage du dispositif correspondant à la figure 1b ;
la figure 6 représente une vue partielle en perspective du dispositif assemblé ;
la figure 7 représente une vue schématique en coupe selon un plan vertical longitudinal passant approximativement au milieu du dispositif du dispositif d'éjection assemblé ;
la figure 8 est une vue partielle en perspective arrière d'une partie du dispositif d'éjection selon le mode de réalisation de l'invention ;
la figure 9 est une vue du dispositif d'éjection selon le mode de réalisation de l'invention en perspective avant coupée selon un plan vertical longitudinal passant approximativement au milieu du dispositif ;
la figure 10 représente le fonctionnement du dispositif d'éjection selon le mode de réalisation de l'invention par une vue schématique en coupe selon un plan vertical longitudinal passant approximativement au milieu du dispositif.

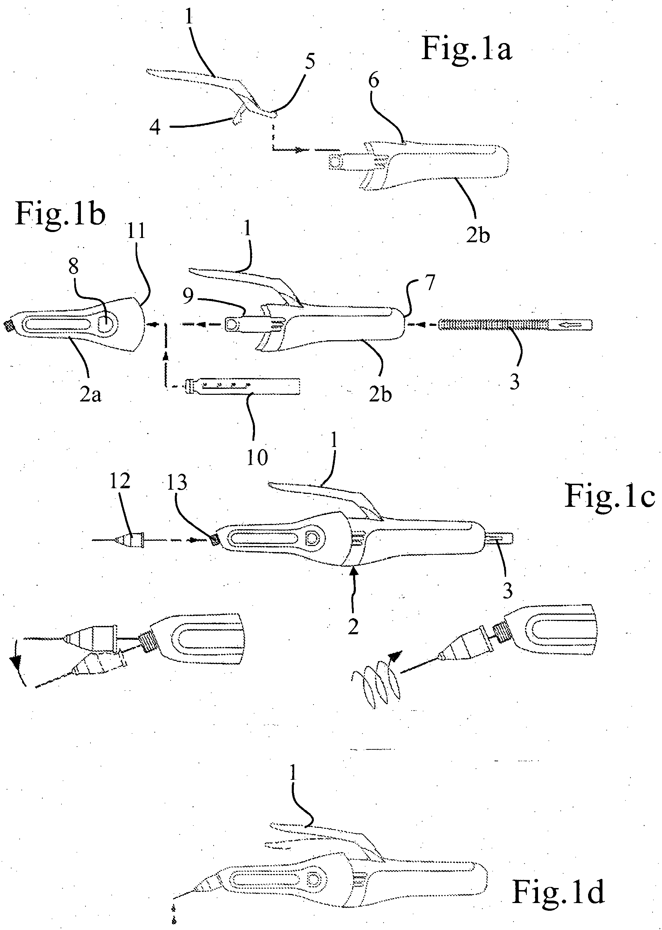

Selon le mode d'exécution préféré, le dispositif d'éjection selon l'invention repose sur l'assemblage de quatre principaux éléments distincts, de forme permettant leur fabrication en matériau plastique par simple injection, et dont l'assemblage peut se faire très simplement selon les étapes illustrées sur les figures 1a à 1d.

Dans une première étape illustrée sur la figure 1a, un levier 1 est assemblé avec un corps arrière 2b. Le levier 1 comprend une partie supérieure horizontale destinée à être manipulée et une extrémité inférieure en forme d'axe 5 qui est positionnée dans un emplacement 6 correspondant du corps arrière 2b. Un cliquet 4 est de plus lié au levier 1 selon une structure monolithique et s'étend vers le bas, vers l'intérieur du corps 2b.

Dans une seconde étape illustrée sur la figure 1 b, la partie avant du corps du dispositif, c'est à dire le porte-conteneur 2a, vient s'assembler au niveau de l'extrémité avant de l'ensemble obtenu précédemment par la coopération de clips 9 du corps arrière 2b se logeant dans des ouvertures correspondantes 8 du porte-conteneur 2a, de sorte de former un corps complet 2. Avant cette opération, il convient de positionner la cartouche 10 contenant le produit à éjecter au sein du porte-conteneur 2a. En variante, ce produit peut déjà être disposé dans le porte-conteneur auparavant, éventuellement directement intégré au sein du porte-conteneur, sans l'intermédiaire d'une cartouche 10 distincte. Dans le même temps, un cylindre de poussée 3 présentant des dents, que nous appellerons crémaillère 3 par la suite, est insérée par une ouverture 7 pratiquée à l'arrière du corps arrière 2b et positionnée dans un alésage cylindrique prévu à cet effet dans la partie centrale du corps 2.

Selon la figure 1 c, un porte-outil 12 avec une aiguille est reliée à l'extrémité 13 du porte-conteneur 2a munie d'un orifice pour l'éjection du produit. Avantageusement, cette liaison pourra se faire selon l'enseignement du document WO03/082387. Pour cela, la forme antérieure du porte-conteneur 2a présente une forme globalement inclinée par rapport au corps 2 mais présentant une surface ayant des génératrices sensiblement parallèles à l'axe de ce corps 2 afin de permettre une insertion droite de l'aiguille dans cette direction avant sa flexion et fixation en position de travail. Le dispositif d'éjection est alors prêt à l'emploi, qui est illustré en figure 1 d par le déplacement vers le bas du levier 1 autour de son axe de rotation 5, entraînant l'éjection du produit contenu dans la cartouche sous la pression de la surface avant de la crémaillère 3. En variante, le porte-outil 12 peut être intégré au porte-conteneur 2a.

La géométrie et la fonction technique des différents éléments du dispositif vont maintenant être détaillés.

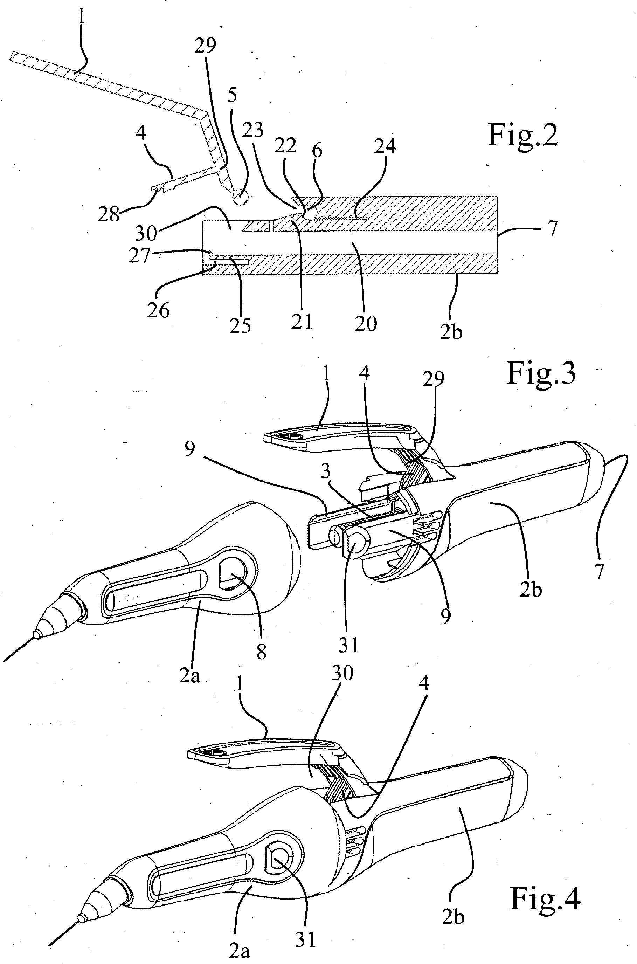

La figure 2 représente plus particulièrement la liaison entre le levier 1 et le corps arrière 2b et permet d'observer en détail les éléments mis en oeuvre lors de la phase illustrée sur la figure 1a. Le levier 1 comprend une extrémité 5 cylindrique, formant son axe de rotation, qui vient se loger dans un emplacement cylindrique 6 de diamètre correspondant formé dans la partie supérieure du corps arrière 2b du dispositif. Cet emplacement cylindrique 6 comprend dans sa partie inférieure une lame élastique longitudinale 21 intégrée au corps 2b, formée par une découpe 24 d'une partie de la surface supérieure de l'alésage cylindrique central 20 du corps arrière 2b. L'axe 5 est d'abord positionné sur deux rails latéraux non représentés réalisés dans la partie supérieure du corps 2, puis déplacé vers l'arrière en glissant sur ces rails le conduisant vers l'ouverture 23 de l'emplacement 6 final, ouverture de plus petite dimension que le diamètre de l'axe 5, la lame 21 s'effaçant vers le bas au sein de l'alésage 20 sous la pression de l'axe 5 du levier 1 pour le laisser passer et atteindre son emplacement 6 par cette ouverture 23. La lame 21 reprend ensuite sa position naturelle grâce à son élasticité et vient épouser une partie inférieure de la circonférence de l'axe 5.

Le levier 1 est de plus relié à un cliquet 4 sensiblement perpendiculaire au levier en une zone de liaison 29. Ce cliquet 4 se termine par une extrémité 28 comprenant une ou plusieurs dents pour coopérer avec une crémaillère comme cela sera détaillé par la suite. Le cliquet 4 et le reste du levier 1 forment une structure monolithique, obtenue par une seule étape d'injection. Leur zone de liaison 29 est telle que le cliquet 4 est mobile élastiquement par rapport au levier 1, selon une rotation autour de sa liaison 29 avec le levier. Pour cela, des découpes ou des zones de moindre épaisseur sont prévues au sein du matériau plastique dans la zone de liaison 29, de sorte de former une zone de moindre rigidité, déformable par rapport au reste du levier. Ces découpes peuvent par exemple dessiner un axe s'étendant sur la largeur du levier dans la zone 29, et relié au cliquet 4 pour représenter son axe de rotation.

Enfin, le corps arrière 2b présente au moins un cliquet anti-retour 25, sous la forme d'une lame élastique longitudinale obtenue par une découpe 26 dans le corps lui-même et formée ainsi lors de l'injection du corps 2b, et présentant une extrémité 27 en forme de pointe complémentaire des dents de la crémaillère 3, s'étendant au sein de l'alésage 20, pour coopérer avec ces dents comme cela sera détaillé par la suite.

Lorsque le levier est en position dans l'emplacement 6 du corps 2b, le porte-conteneur 2a est assemblé au corps 2b alors qu'une crémaillère 3 est insérée dans l'alésage cylindrique longitudinal 20 du corps ainsi assemblé par une ouverture arrière 7, jusqu'à venir en butée contre la cartouche 10 contenant le produit à éjecter. La figure 3 illustre cette étape correspondant à celle de la figure 1 b, dans une configuration intermédiaire dans laquelle la crémaillère 3 est déjà insérée alors que le porte-conteneur 2a s'apprête à l'être.

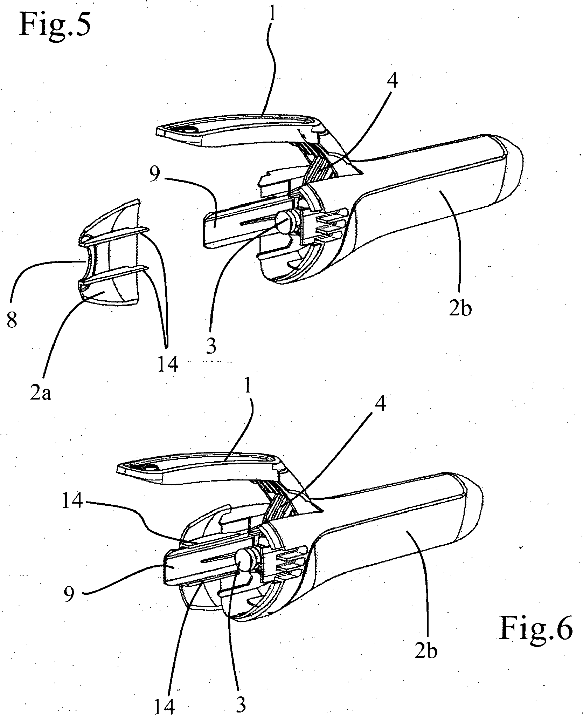

Pour réaliser cet assemblage, le corps arrière 2b présente deux lames latérales 9 s'étendant longitudinalement vers l'avant, aptes à coopérer avec des rails complémentaires 14 inférieur et supérieur prévus contre les parois latérales à l'intérieur du porte-conteneur 2a, comme cela est illustré sur les figures 5 et 6. L'extrémité de ces lames 9 présente une protubérance 31 orientée vers l'extérieur, destinée à coopérer avec des ouvertures correspondantes 8 du porte-conteneur 2a. Lors de l'insertion des lames 9 dans les rails 14 du porte-conteneur 2a, les protubérances 31 viennent frotter contre les parois latérales intérieures du porte-conteneur 2a, en provoquant une déformation élastique des lames 9 vers l'intérieur du corps 2. Dès que les protubérances 31 atteignent les ouvertures 8 de forme correspondante, elles y prennent place sous l'effet élastique des lames 9 qui retrouvent leur écartement normal initial et leur direction longitudinale et parallèle. La forme allongée des lames 9 maintenues sur leur longueur par les rails 14 inférieur et supérieur permet une liaison rigide et sans jeu gênant entre les deux parties 2a et 2b du corps pour supporter ainsi efficacement les efforts subis lors de son utilisation. Toute autre liaison mécanique équivalente entre ces deux parties pourrait convenir.

D'autre part, l'insertion de la crémaillère 3 dans l'alésage 20 du corps 2 la conduit en appui sur la surface inférieure de la lame 21 d'insertion du levier 1, empêchant ensuite tout mouvement vers le bas de cette lame 21. Par ce biais, l'axe 5 du levier 1 se trouve verrouillé dans l'emplacement 6 du corps 2, ne pouvant plus s'échapper par l'ouverture 23 de plus petite dimension.

Selon une solution avantageuse, la partie supérieure de la lame 21 forme d'une part une pente douce vers le haut guidant l'axe 5 du levier 1 vers l'ouverture 23 pour favoriser l'étape d'assemblage avec le corps 2, explicitée précédemment, puis présente une surface arrondie 22 vers le bas correspondant à la courbure de l'axe 5 pour former une surface d'accueil inférieure de l'emplacement 6 de l'axe 5. En remarque, si l'axe 5 du levier 1 n'est pas parfaitement positionné au fond de son emplacement 6 lors de l'étape initiale d'assemblage représentée à la figure 1 a, mais reste par exemple bloqué dans une zone intermédiaire en appuyant sur la lame 21 alors déplacée vers le bas, l'insertion de la crémaillère 3 dans l'alésage 20 induit une force de poussée vers le haut sur la lame 21 qui entraîne finalement le glissement de l'axe 5 sur sa pente 22 jusqu'à la position finale de cet axe 5 dans laquelle il occupe tout l'espace 6 et une position finale horizontale de la lame 21. L'insertion de la crémaillère 3 remplit ainsi une fonction supplémentaire de positionnement final du levier 1, garantissant ainsi son bon positionnement, avant de le verrouiller dans cette position.

Selon une réalisation avantageuse du dispositif, la cartouche insérée dans le porte-conteneur 2a vient jusqu'aux abords des lames 9 au niveau des ouvertures 8 de sorte que le mouvement inverse du corps arrière 2b, qui nécessiterait une nouvelle déformation des lames 9 en appuyant sur les protubérances 31 pour les extraire des ouvertures 8 tout en tirant le corps 2b vers l'arrière, est rendu difficile, voire impossible. Cette géométrie gêne ainsi cet échappement des protubérances 31 et permet un verrouillage ou quasi-verrouillage du corps 2 ainsi assemblé, induisant un verrouillage de la crémaillère 3 au sein de l'alésage cylindrique central 20, induisant de même le verrouillage du levier 1, comme expliqué cidessus. Le dispositif ainsi assemblé n'est donc pas ou très difficilement démontable et garantit un positionnement sûr et performant dés différents éléments.

Les figures 6 à 9 illustrent de différentes manières le dispositif ainsi assemblé.

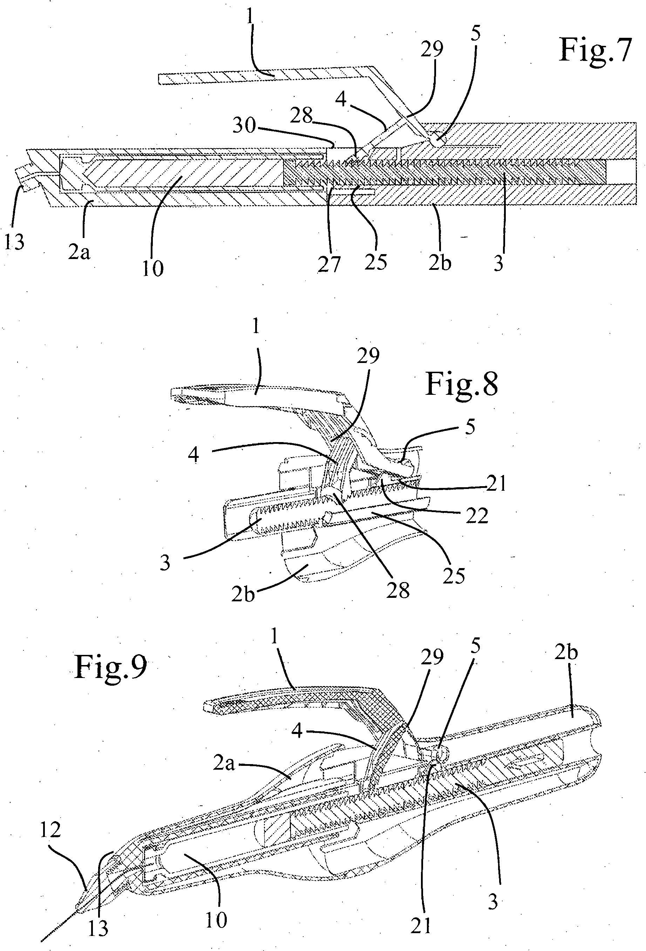

L'extrémité dentée 28 du cliquet 4 du dispositif d'éjection, particulièrement visible sur les figures 7, 8 et 9, pénètre dans une ouverture supérieure 30 du corps 2 pour venir dans une zone proche des dents de la crémaillère 3, et la partie extrême 27 du cliquet anti-retour 25 vient se loger dans une dent de la crémaillère. L'insertion de la crémaillère 3 dans l'alésage 20 s'accompagne d'un clic sonore lorsque l'extrémité 27 du cliquet anti-retour 25 prend position entre deux dents de la crémaillère. Comme cette extrémité 27 du cliquet anti-retour 25 occupe une position légèrement plus avancée que celle de l'extrémité 28 du cliquet 4, ce clic sonore intervient quand la crémaillère est suffisamment avancée pour pouvoir être actionnée par le levier 1 et ce clic permet d'informer l'utilisateur que la crémaillère est suffisamment avancée.

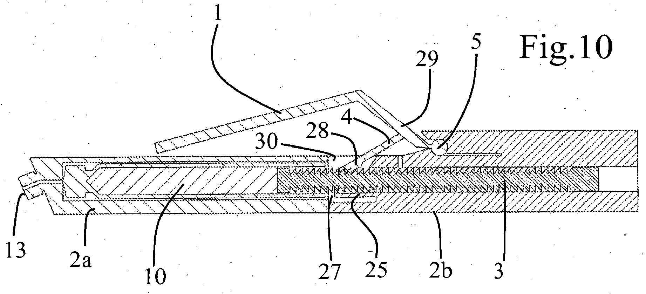

La figure 10 illustre le fonctionnement du dispositif d'éjection. Le levier 1 est pressé vers le bas contre le porte-conteneur 2a par une rotation autour de l'axe 5, entraînant une déformation élastique du cliquet 4 en appui sur la crémaillère 3 autour de sa zone de liaison 29 avec le levier, grâce à la propriété de flexibilité du matériau utilisé et la géométrie choisie au niveau notamment de la liaison 29 entre le cliquet 4 et le levier 1. Ce mouvement du cliquet 4 permet à son extrémité dentée 28 qui coopère avec des dents de la crémaillère 3, d'induire une force de poussée sur la crémaillère et son avancée au sein de l'alésage 20. Dans le mode de réalisation proposé, l'extrémité 28 du cliquet 4 présente une partie arrondie épousant la forme cylindrique supérieure de la crémaillère 3 et trois rangées de dents pour coopérer en même temps avec trois dents de la crémaillère 3 et garantir une bonne coopération entre ces deux éléments 3, 4.

Lorsque le levier est relâché, le cliquet 4 exerce un effort de rappel élastique vers sa position initiale et entraîne le levier 1 vers sa position haute initiale. Dans cette phase, le cliquet anti-retour 25 empêche la crémaillère de revenir en arrière. Pour cela, son extrémité 27 et la forme des dents de la crémaillère présentent une pente leur permettant de glisser dans le sens de l'avancée de la crémaillère en entraînant une déformation vers le bas du cliquet 25, jusqu'à ce que la dent de la crémaillère lui échappe, ce qui entraîne alors le repositionnement élastique du cliquet anti-retour dans la dent suivante, accompagné d'un clic sonore qui confirme à l'utilisateur que la crémaillère avance. En revanche, la surface antérieure de l'extrémité 27 du cliquet anti-retour 25 présente une surface sensiblement verticale, coopérant avec une surface verticale correspondante des dents de la crémaillère, créant un blocage empêchant tout mouvement vers l'arrière de la crémaillère. L'avancée de la crémaillère 3 sert à pousser un piston en appui sur le volume 10 portant le contenu à éjecter.

Selon une réalisation avantageuse du dispositif, le levier 1 et le cliquet 4 sont en matériau plastique injecté. Ainsi, lors de l'actionnement du levier, le cliquet 4 commence par se déformer élastiquement en exerçant une force de plus en plus forte sur la crémaillère, jusqu'à ce que cette dernière avance finalement dans un second temps. Ce comportement en deux temps permet d'atténuer l'effet d'une pression plus ou moins forte sur le levier et d'entraîner une éjection relativement constante et douce du produit, améliorant ainsi le bien-être du patient dans le cas d'une seringue. Une partie d'une trop forte force de pression sur le levier sera absorbée par la déformation des éléments du dispositif.

Comme cela a été exposé précédemment, un procédé de fabrication rapide et peu coûteux du dispositif d'éjection consiste à fabriquer séparément par injection de matériau plastique les quatre différents composants essentiels du dispositif, le levier 1, le porte-conteneur 2a, le corps arrière 2b et la crémaillère 3. Le matériau utilisé peut être du polyamide, du polypropylène, de l'ABS ou tout autre matériau plastique. Un plastique recyclable sera bien adapté au dispositif qui est jetable après utilisation. Un matériau plastique transparent peut aussi être avantageusement utilisé, notamment pour le porte-conteneur 2a, pour permettre de visualiser le volume restant de produit à éjecter. Une dernière étape du procédé de fabrication concerne ensuite le procédé d'assemblage de ces composants. Toutefois, pour faciliter le transport et la distribution du dispositif, il est intéressant de prévoir sa distribution dans un état non assemblé pour un assemblage sur place juste avant l'utilisation. Cette solution est rendue possible par la facilité de l'assemblage des composants, décrite en référence avec les figures 1 a à 1d. Cela permet d'autre part à l'utilisateur final de pouvoir choisir le produit qu'il souhaite injecter.

En variante, les composants précédents peuvent être fabriqués différemment et dans d'autres matériaux. La crémaillère 3 peut par exemple être réalisée en acier inoxydable.

Un deuxième mode de réalisation du dispositif selon l'invention, non représenté, pourrait être obtenu sur Ja base de la figure 7 du document WO2005/007224, différant du mode d'exécution précédent en ce que l'alésage 20 présente un épaulement pour recevoir une chemise réalisée par exemple en polytétrafluoroéthylène ou un autre matériau plastique tel qu'un polyétheréthercétone (PEEK).

Un tel dispositif d'éjection peut être utilisé dans le domaine médical pour l'injection de produits tels que des anesthésiques dans des tissus durs ou pour le dépôt de colles, de résines ou d'amalgames. Par exemple, une implémentation pour une seringue anesthésiante dans le domaine dentaire est très intéressante. Il peut également être utilisé dans le domaine paramédical pour déposer des quantités déterminées de collagène. Il peut en outre être utilisé dans le domaine de la micromécanique et de la bijouterie pour effectuer des collages ou des microsoudures ou encore pour déposer des produits.

Finalement, la solution atteint bien les objets recherchés et présente les avantages suivants :
- la présence d'un levier parfaitement fixé de manière verrouillée permet d'atteindre une éjection performante ;
- le dispositif repose sur une structure simple avec peu d'éléments, sans ressort ni axe distinct et indépendant, réalisables facilement et à bas coûts, par des procédés compatibles avec l'emploi de matériaux recyclables : le procédé de fabrication est donc économique et compatible avec un concept de dispositif jetable, ce qui élimine les problèmes de nettoyage ;
- la composition du dispositif en peu d'éléments faciles à assembler permet sa distribution facile, son montage à toute étape même au moment du besoin d'une injection.

Le dispositif selon la présente invention a été conçu sur la base du concept de dispositif jetable. Toutefois, son utilisation non jetable, en utilisant les procédés de nettoyages de l'art antérieur, ne sortirait pas pour autant du cadre de l'invention. En effet, la simplicité du dispositif et la possibilité de le rendre démontable, par exemple en modifiant la liaison entre les corps 2a et 2b pour faciliter l'échappement des bras 9, peut permettre son implémentation avantageuse pour une utilisation démontable pour son nettoyage, simplifié du fait de la simplification de la structure du dispositif.

De plus, le mode d'exécution préféré a été décrit précédemment. D'autres modes d'exécution de l'invention sont possibles, en ne reprenant que certains des éléments très simplifiés du dispositif parmi lesquels l'ensemble levier 1 - cliquet 4, le dispositif d'assemblage du levier sur le corps 2, le corps composé de deux parties principales 2a, 2b, le cliquet anti-retour 25,..., ce qui permettrait déjà d'obtenir un dispositif d'éjection amélioré, simplifié et moins coûteux.

## Revendications

1. Dispositif d'éjection d'un produit liquide ou pâteux, comprenant un corps (2) ayant une partie avant (2a) destinée à contenir le produit à éjecter et munie d'un orifice (13) pour l'éjection, du produit, une crémaillère (3) se déplaçant dans un alésage (20) d'une partie arrière (2b) du corps (2) et faisant varier le volume de la partie avant (2a) et un mécanisme de déplacement de la crémaillère (3) comprenant un levier articulé (1) autour d'un premier axe de rotation et un cliquet (4) agissant sur ladite crémaillère (3), **caractérisé en ce que** le cliquet (4) et le levier (1) forment une structure monolithique en matériau plastique et **en ce qu'**une zone de liaison (29) entre le cliquet (4) et le levier est déformable de façon à permettre un mouvement élastique relatif du cliquet (4) et du levier (1).

2. Dispositif d'éjection d'un produit liquide ou pâteux selon la revendication 1, **caractérisé en ce que** le levier (1) comprend une extrémité cylindrique (5) dont l'axe coïncide avec l'axe de rotation du levier (1) et qui vient se loger dans un logement cylindrique (6) de diamètre correspondant formé dans la partie arrière (2b) dudit corps (2).

3. Dispositif d'éjection d'un produit liquide ou pâteux selon la revendication 2, **caractérisé en ce que** l'extrémité cylindrique (5) du levier (1) forme une seule structure monolithique avec le levier (1) et le cliquet (4).

4. Dispositif d'éjection d'un produit liquide ou pâteux selon l'une des revendications précédentes, **caractérisé en ce que** le cliquet (4) est sensiblement perpendiculaire au levier (1) dans leur zone de liaison (29) en définissant un second axe de rotation du cliquet (4) relativement au levier.

5. Dispositif d'éjection d'un produit liquide ou pâteux selon l'une des revendications précédentes, **caractérisé en ce que** la zone de liaison déformable (29) présente des découpes et/ou des zones de moindre épaisseur de façon à réduire sa rigidité.

6. Dispositif d'éjection d'un produit liquide ou pâteux selon l'une des revendications précédentes, **caractérisé en ce que** le cliquet (4) présente une flexibilité apte à une éjection en deux temps, un premier temps consistant en sa déformation sans éjection et un appui de plus en plus fort sur la crémaillère (3) et un second temps consistant en l'avancée de la crémaillère (3) et l'éjection du produit.

7. Dispositif d'éjection d'un produit liquide ou pâteux selon l'une des revendications précédentes, **caractérisé en ce qu'**il comprend un moyen de verrouillage de la liaison entre le levier (1) et le corps (2).

8. Dispositif d'éjection d'un produit liquide ou pâteux selon l'une des revendications précédentes, **caractérisé en ce que** le corps (2) comprend au moins une lame élastique (21) permettant de clipser l'extrémité (5) du levier (1) dans le logement (6) du corps (2).

9. Dispositif d'éjection d'un produit liquide ou pâteux selon la revendication 8, **caractérisé en ce que** la lame élastique (21) est intégrée au corps (2), au niveau de la surface supérieure de l'alésage (20), de sorte de pouvoir se déformer au sein de cet alésage (20) pour l'assemblage du levier (1) sur le corps (2) et de ne plus pouvoir se déformer et verrouiller le levier (1) sur le corps (2) lorsque la crémaillère (3) est présente dans l'alésage (20).

10. Dispositif d'éjection d'un produit liquide ou pâteux selon la revendication 8 ou 9, **caractérisé en ce que** la lame élastique (21) présente une surface supérieure arrondie (22) épousant l'extrémité (5) et assurant son bon positionnement dans le logement (6) lors de l'insertion de la crémaillère (3).

11. Dispositif d'éjection d'un produit liquide ou pâteux selon l'une des revendications précédentes, **caractérisé en ce qu'**il comprend une lame élastique (25) intégrée au corps (2) remplissant la fonction de cliquet anti-retour.

12. Dispositif d'éjection d'un produit liquide ou pâteux selon la revendication 11, **caractérisé en ce qu'**une extrémité (27) du cliquet anti-retour se trouve positionnée en avant ou au même niveau qu'une extrémité (28) du cliquet (4) lié au levier (1).

13. Dispositif d'éjection d'un produit liquide ou pâteux selon l'une des revendications précédentes, **caractérisé en ce que** la partie avant (2a) du corps (2) forme au moins un porte-conteneur qui est assemblé à la partie arrière (2b) dudit corps recevant la crémaillère (3).

14. Dispositif d'éjection d'un produit liquide ou pâteux selon la revendication 13, **caractérisé en ce que** la partie avant (2a) du corps (2) et la partie arrière (2b) sont reliées par au moins deux lames élastiques longitudinales (9) portées par ladite partie arriere (2b) et positionnées dans des rails (14) de ladite partie avant (2a).

15. Dispositif d'éjection d'un produit liquide ou pâteux selon la revendication 14, **caractérisé en ce que** les lames (9) présentent une protubérance (31) à leur extrémité pouvant se clipser dans des ouvertures (8) de l'autre partie.

16. Dispositif d'éjection d'un produit liquide ou pâteux selon la revendication précédente, **caractérisé en ce que** la liaison entre les deux parties avant (2a) et arrière (2b) du corps (2) est verrouillée ou quasi-verrouillée, entraînant le verrouillage de l'ensemble des composants assemblés pour une unique utilisation du dispositif qui est jetable.

17. Dispositif d'éjection d'un produit liquide ou pâteux selon l'une des revendications 1-15, **caractérisé en ce que** la liaison entre les deux parties avant et arrière (2a, 2b) du corps (2) est démontable pour permettre le désassemblage du dispositif et son nettoyage.

18. Dispositif d'éjection d'un produit liquide ou pâteux selon l'une des revendications précédentes, **caractérisé en ce qu'**il est composé de quatre parties principales destinées à être assemblées, un levier (1) incluant un cliquet (4), une partie avant (2a) de corps (2) formant porte-conteneur, une partie arrière (2b) de corps (2) et une crémaillère (3).

19. Dispositif d'éjection d'un produit liquide ou pâteux selon la revendication précédente, **caractérisé en ce qu'**au moins le levier (1), ladite partie avant (2a) et ladite partie arrière (2b) sont en matériau plastique injecté.

20. Dispositif d'éjection d'un produit liquide ou pâteux selon l'une des revendications précédentes, **caractérisé en ce qu'**au moins le porte-conteneur est en matériau plastique transparent.

21. Dispositif d'éjection d'un produit liquide ou pâteux selon la revendication précédente, **caractérisé en ce que** le corps (2) présente une extrémité antérieure (13) pour recevoir un porte-outil (12) avec une aiguille, cette extrémité antérieure ayant une forme inclinée par rapport au corps (2) et présentant des génératrices parallèles au corps (2) pour permettre une insertion droite de l'aiguille.

22. Dispositif d'éjection selon l'une des revendications précédentes, **caractérisé en ce qu'**il comprend une chemise dans la partie arrière (2b).

23. Procédé de fabrication d'un dispositif d'éjection d'un produit liquide ou pâteux tel que défini dans la revendication 1 **caractérisé en ce qu'**il comprend une étape de fabrication d'au moins quatre éléments distincts, un levier (1) intégrant un cliquet (4), le cliquet (4) et le levier (1) étant reliés par une zone (29) déformable élastiquement et formant une structure monolithique en matériau plastique, un corps (2) comprenant une partie avant (2a) formant porte-conteneur et une partie arrière (2b) recevant une crémaillère (3) et **en ce qu'**il comprend une phase d'assemblage de ces éléments comprenant les étapes suivantes :
a - insertion du levier sur la partie arrière (2b) du corps (2) en engageant par clipsage son extrémité arrière cylindrique (5) dans un logement cylindrique (6) de diamètre correspondant formé dans la partie arrière (2b) dudit corps (2),
b - insertion de la crémaillère (3) au sein d'un alésage (20) de la partie arrière (2b) et,
c - liaison du porte-conteneur à la partie arrière (2b) du corps (2).

24. Procédé de fabrication d'un dispositif d'éjection selon la revendication précédente, **caractérisé en ce qu'**il comprend au moins trois étapes d'injection de matériau plastique pour former trois parties distinctes du dispositif, un levier (1) intégrant un cliquet (4), une partie avant (2a) formant porte-conteneur et une partie arrière (2b) recevant la crémaillère (3).

25. Procédé de fabrication d'un dispositif d'éjection d'un produit liquide ou pâteux selon la revendication précédente, **caractérisé en ce qu'**il comprend une quatrième étape d'injection en matériau plastique pour former ladite crémaillère (3).

## Patentansprüche

1. Auswurfvorrichtung eines flüssigen oder pastösen Produkts, umfassend einen Körper (2) mit einem Vorderteil (2a), der bestimmt ist, das auszuwerfende Produkt zu enthalten und mit einer Öffnung (13) für den Auswurf des Produkts ausgestattet ist, wobei sich eine Zahnstange (3) in einer Bohrung (20) eines Hinterteils (2b) des Körpers (2) verlagert und das Volumen des Vorderteils (2a) verändert, und einen Verlagerungsmechanismus der Zahnstange (3), umfassend einen Hebel (1), der um eine erste Rotationsachse angelenkt ist, und eine Sperre (4), die auf die Zahnstange (3) einwirkt, **dadurch gekennzeichnet, dass** die Sperre (4) und der Hebel (1) eine monolithische Struktur aus Kunststoffmaterial bilden und dass eine Verbindungszone (29) zwischen der Sperre (4) und dem Hebel (4) derart verformbar ist, dass eine relative elastische Bewegung der Sperre (4) und des Hebels (1) gestattet ist.

2. Auswurfvorrichtung eines flüssigen oder pastösen Produkts nach Anspruch 1, **dadurch gekennzeichnet, dass** der Hebel (1) ein zylindrisches Ende (5) umfasst, dessen Achse mit der Rotationsachse des Hebels (1) zusammenfällt und das in einer zylindrischen Aufnahme (6) mit einem entsprechenden Durchmesser ruht, die im Hinterteil (2b) des Körpers (2) ausgebildet ist.

3. Auswurfvorrichtung eines flüssigen oder pastösen Produkts nach Anspruch 2, **dadurch gekennzeichnet, dass** das zylindrische Ende (5) des Hebels (1) mit dem Hebel (1) und der Sperre (4) eine einzige monolithische Struktur bildet.

4. Auswurfvorrichtung eines flüssigen oder pastösen Produkts nach einem der vorangehenden Ansprüche, **dadurch gekennzeichnet, dass** in ihrer Verbindungszone (29) die Sperre (4) etwa senkrecht zum Hebel (1) ist und dabei eine zweite Rotationsachse der Sperre (4) in Bezug zum Hebel definiert wird.

5. Auswurfvorrichtung eines flüssigen oder pastösen Produkts nach einem der vorangehenden Ansprüche, **dadurch gekennzeichnet, dass** die verformbare Verbindungszone (29) Ausschnitte und/oder Zonen geringerer Dicke aufweist, um ihre Solidität zu verringern.

6. Auswurfvorrichtung eines flüssigen oder pastösen Produkts nach einem der vorangehenden Ansprüche, **dadurch gekennzeichnet, dass** die Sperre (4) eine Flexibilität aufweist, die für einen Auswurf in zwei Etappen geeignet ist, wobei eine erste Etappe in ihrer Verformung ohne Auswurf und einer immer stärkeren Abstützung auf der Zahnstange (3) besteht und eine zweite Etappe aus dem Vorschub der Zahnstange (3) und dem Auswurf des Produkts besteht.

7. Auswurfvorrichtung eines flüssigen oder pastösen Produkts nach einem der vorangehenden Ansprüche, **dadurch gekennzeichnet, dass** sie ein Verriegelungsmittel der Verbindung zwischen dem Hebel (1) und dem Körper (2) umfasst.

8. Auswurfvorrichtung eines flüssigen oder pastösen Produkts nach einem der vorangehenden Ansprüche, **dadurch gekennzeichnet, dass** der Körper (2) mindestens eine elastische Zunge (21) umfasst, die erlaubt, das Ende (5) des Hebels (1) in die Aufnahme (6) des Körpers (2) zu klipsen.

9. Auswurfvorrichtung eines flüssigen oder pastösen Produkts nach Anspruch 8, **dadurch gekennzeichnet, dass** die elastische Zunge (21) im Bereich der oberen Fläche der Bohrung (20) in den Körper (2) integriert ist, so dass sie innerhalb dieser Bohrung (20) für die Montage des Hebels (1) auf dem Körper (2) verformbar ist und nicht mehr verformbar ist und den Hebel (1) nicht mehr auf dem Körper (2) verriegeln kann, wenn die Zahnstange (3) in der Bohrung (20) vorhanden ist.

10. Auswurfvorrichtung eines flüssigen oder pastösen Produkts nach Anspruch 8 oder 9, **dadurch gekennzeichnet, dass** die elastische Zunge (21) eine abgerundete obere Fläche (22) aufweist, die sich an das Ende (5) anschmiegt und seine gute Positionierung in der Aufnahme (6) beim Einsetzen der Zahnstange (3) sichert.

11. Auswurfvorrichtung eines flüssigen oder pastösen Produkts nach einem der vorangehenden Ansprüche, **dadurch gekennzeichnet, dass** sie eine in den Körper (2) integrierte elastische Zunge (25) umfasst, die die Funktion einer Anti-Rücklauf-Sperre erfüllt.

12. Auswurfvorrichtung eines flüssigen oder pastösen Produkts nach Anspruch 11, **dadurch gekennzeichnet, dass** ein Ende (27) der Anti-Rücklauf-Sperre vor oder im selben Bereich wie ein Ende (28) der mit dem Hebel (1) verbundenen Sperre (4) positioniert ist.

13. Auswurfvorrichtung eines flüssigen oder pastösen Produkts nach einem der vorangehenden Ansprüche, **dadurch gekennzeichnet, dass** der Vorderteil (2a) des Körpers (2) mindestens einen Behälterhalter bildet, der am Hinterteil (2b) des Körpers, welcher die Zahnstange (3) aufnimmt, montiert ist.

14. Auswurfvorrichtung eines flüssigen oder pastösen Produkts nach Anspruch 13, **dadurch gekennzeichnet, dass** der Vorderteil (2a) des Körpers (2) und der Hinterteil (2b) anhand von mindestens zwei länglichen elastischen Zungen (9) verbunden sind, die von dem Hinterteil (2b) getragen werden und in Schienen (14) des Vorderteils (2a) positioniert sind.

15. Auswurfvorrichtung eines flüssigen oder pastösen Produkts nach Anspruch 14, **dadurch gekennzeichnet, dass** die Zungen (9) an ihrem Ende einen Vorsprung (31) aufweisen, der in Öffnungen (8) des anderen Teils klipsbar ist.

16. Auswurfvorrichtung eines flüssigen oder pastösen Produkts nach vorangehendem Anspruch, **dadurch gekennzeichnet, dass** die Verbindung zwischen den zwei Teilen, dem Vorderteil (2a) und dem Hinterteil (2b) des Körpers (2), verriegelt oder quasi verriegelt ist, was die Verriegelung der Gesamtheit der montierten Bauteile für eine einzige Verwendung der Vorrichtung, die eine Einwegvorrichtung ist, bewirkt.

17. Auswurfvorrichtung eines flüssigen oder pastösen Produkts nach einem der Ansprüche 1 - 15, **dadurch gekennzeichnet, dass** die Verbindung zwischen den zwei Teilen, dem Vorder- und Hinterteil (2a, 2b) des Körpers (2), demontierbar ist, um die Demontage der Vorrichtung und ihre Reinigung zu erlauben.

18. Auswurfvorrichtung eines flüssigen oder pastösen Produkts nach einem der vorangehenden Ansprüche, **dadurch gekennzeichnet, dass** sie aus vier zur Montage bestimmten Hauptteilen, einem Hebel (1), zu dem eine Sperre gehört, (4), einem Vorderteil (2a) des Körpers (2), der einen Behälterhalter bildet, einem Hinterteil (2b) des Körpers (2) und einer Zahnstange (3), zusammengesetzt ist.

19. Auswurfvorrichtung eines flüssigen oder pastösen Produkts nach vorangehendem Anspruch, **dadurch gekennzeichnet, dass** mindestens der Hebel (1), der Vorderteil (2a) und der Hinterteil (2b) aus Spritzgusskunststoff sind.

20. Auswurfvorrichtung eines flüssigen oder pastösen Produkts nach einem der vorangehenden Ansprüche, **dadurch gekennzeichnet, dass** mindestens der Behälterhalter aus durchsichtigem Kunststoffmaterial ist.

21. Auswurfvorrichtung eines flüssigen oder pastösen Produkts nach vorangehendem Anspruch, **dadurch gekennzeichnet, dass** der Körper (2) ein vorderes Ende (13) zur Aufnahme eines Werkzeughalters (12) mit einer Nadel aufweist, wobei dieses vordere Ende eine im Verhältnis zum Körper (2) geneigte Form hat und Mantellinien aufweist, die parallel zum Körper (2) sind, um ein gerades Einsetzen der Nadel zu erlauben.

22. Auswurfvorrichtung nach einem der vorangehenden Ansprüche, **dadurch gekennzeichnet, dass** sie im Hinterteil (2b) eine Hülle umfasst.

23. Herstellungsverfahren einer Auswurfvorrichtung eines flüssigen oder pastösen Produkts nach Anspruch 1, **dadurch gekennzeichnet, dass** es einen Herstellungsschritt von mindestens vier unterschiedlichen Elementen, eines Hebels (1), zu dem eine Sperre (4) gehört, wobei die Sperre (4) und der Hebel mittels einer elastisch verformbaren Zone (29) verbunden sind und eine monolithische Struktur aus Kunststoffmaterial bilden, eines Körpers, der einen Vorderteil (2a), der einen Behälterhalter bildet, und einen Hinterteil (2b), der eine Zahnstange (3) aufnimmt, umfasst, und dass es eine Montagephase dieser Elemente umfasst, welche die folgenden Schritte umfasst:
a - Einsetzen des Hebels auf den Hinterteil (2b) des Körpers (2) durch Einführen seines zylindrischen hinteren Endes (5) durch Klipsen in eine zylindrische Aufnahme (6) mit einem entsprechenden Durchmesser, die im Hinterteil (2b) des Körpers (2) ausgebildet ist,
b - Einsetzen der Zahnstange (3) in eine Bohrung (20) des Hinterteils (2b) und
c - Verbinden des Behälterhalters mit dem Hinterteil (2b) des Körpers (2).

24. Herstellungsverfahren einer Auswurfvorrichtung nach vorangehendem Anspruch, **dadurch gekennzeichnet, dass** es mindestens drei Spritzgießschritte von Kunststoffmaterial umfasst, um drei unterschiedliche Teile der Vorrichtung, einen Hebel (1), zu dem eine Sperre gehört, (4), einen Vorderteil (2a), der einen Behälterhalter bildet, und einen Hinterteil (2b), der die Zahnstange (3) aufnimmt, zu bilden.

25. Herstellungsverfahren einer Auswurfvorrichtung eines flüssigen oder pastösen Produkts nach vorangehendem Anspruch, **dadurch gekennzeichnet, dass** es einen vierten Spritzgießschritt von Kunststoffmaterial umfasst, um die Zahnstange (3) zu bilden.

## Claims

1. A device for ejecting a liquid or pasty product, comprising a body (2), having a front portion (2a) which is designed to contain the product to be ejected and is provided with a hole (13) for ejecting the product, a rack (3) which moves along a bore (20) of a rear portion (2b) of the body (2) and varies the volume of the front portion (2a), and a rack (3) movement mechanism comprising a hinged lever (1) around a first rotation axis and a pawl (4) for acting on the rack (3), **characterized in that** the pawl (4) and the lever (1) form a monolithic structure made of plastic and **in that** a connection zone (29) between the pawl (4) and the lever is deformable so as to allow a relative elastic movement of the pawl and the lever.

2. The device for ejecting a liquid or pasty product as claimed in claim 1, **characterized in that** the lever (1) comprises a cylindrical end (5) which axis coincide with the rotation axis of the lever (1) and which lodges itself in a cylindrical seat (6) of corresponding diameter in the rear portion (2b) of said body (2).

3. The device for ejecting a liquid or pasty product as claimed in claim 2, **characterized in that** the cylindrical end (5) of the lever (1) form a single monolithic structure with the lever (1) and the pawl (4).

4. The device for ejecting a liquid or pasty product as claimed in one of the preceding claims, **characterized in that** the pawl (4) is approximately perpendicular to the lever (1) in the connection zone (29) while defining a second rotation axis of the pawl with respect to the lever.

5. The device for ejecting a liquid or pasty product as claimed in one of the preceding claims, **characterized in that** the deformable connection zone (29) has recesses and/or zones of reduced thickness to reduce its stiffness.

6. The device for ejecting a liquid or pasty product as claimed in one of the preceding claims, **characterized in that** the pawl (4) has a flexibility suitable for ejection in two steps, an initial step consisting in its deformation without ejection, with increasing pressure on the rack (3), and a second step consisting in the rack (3) advancing and the product being ejected.

7. The device for ejecting a liquid or pasty product as claimed in one of the preceding claims, **characterized in that** it comprises a means for locking the connection between the lever (1) and the body (2).

8. The device for ejecting a liquid or pasty product as claimed in one of the preceding claims, **characterized in that** the body (2) comprises at least one leaf spring (21) for snap-engaging the end (5) of the lever (1) in the seat (6) of the body (2).

9. The device for ejecting a liquid or pasty product as claimed in claim 8, **characterized in that** the leaf spring (21) is integrated into the body (2), on the upper surface of the bore (20), in such a way that it can deform into this bore (20) for the assembly of the lever (1) onto the body (2) and then no longer be able to deform and to lock the lever (1) to the body (2) when the rack (3) is present inside the bore (20).

10. The device for ejecting a liquid or pasty product as claimed in claim 8 or 9, **characterized in that** the leaf spring (21) has a rounded upper surface (22) which conforms to the end (5) and positions it correctly in the seat (6) when the rack (3) is inserted.

11. The device for ejecting a liquid or pasty product as claimed in one of the preceding claims, **characterized in that** it comprises a leaf spring (25) integrated into the body (2) and fulfilling the function of a non-return pawl.

12. The device for ejecting a liquid or pasty product as claimed in claim 11, **characterized in that** an end (27) of the non-return pawl is positioned forward or at the same level of an end (28) of the pawl (4) connected to the lever (1).

13. The device for ejecting a liquid or pasty product as claimed in one of the preceding claims, **characterized in that** the front portion (2a) of the body (2) forms at least one container holder which is assembled with the rear portion of said body provided with the rack (3).

14. The device for ejecting a liquid or pasty product as claimed in claim 13, **characterized in that** the front portion (2a) of the body (2) and the rear portion (2b) are connected by at least two longitudinal leaf springs (9) held by the rear portion (2b) and positioned in tracks (14) of the front portion (2a).

15. The device for ejecting a liquid or pasty product as claimed in claim 14, **characterized in that** the longitudinal leaf springs (9) have a protuberance (31) at their end able to click in openings (8) in the other portion.

16. The device for ejecting a liquid or pasty product as claimed in the preceding claim, **characterized in that** the connection between the front (2a) and rear (2b) portions of the body (2) is locked or virtually locked, thereby locking all of the assembled components for a single use of the device, which is disposable.

17. The device for ejecting a liquid or pasty product as claimed in one of claims 1 to 15, **characterized in that** the connection between the front and rear portions (2a, 2b) of the body (2) is separable to allow the device to be disassembled for cleaning purposes.

18. The device for ejecting a liquid or pasty product as claimed in one of the preceding claims, **characterized in that** it is made up of four assembled main portions, a lever (1) which incorporates a pawl (4), a front portion (2a) of the body (2) forming a container holder, a rear portion (2b) of the body (2) and a rack (3).

19. The device for ejecting a liquid or pasty product as claimed in the preceding claim, **characterized in that** at least the lever (1), the front portion (2a) and the rear portion (2b) are made of an injection-molded plastic.

20. The device for ejecting a liquid or pasty product as claimed in one of the preceding claims, **characterized in that** at least the container holder is made of a transparent plastic.

21. The device for ejecting a liquid or pasty product as claimed in the preceding claim, **characterized in that** the body (2) has a forward end (13) for accommodating a tool holder (12) with a needle, this forward end having a shape which is inclined relative to the body (2) and has generatrices parallel to the body (2) to enable straight insertion of the needle.

22. The ejection device (1) as claimed in one of the preceding claims, **characterized in that** it includes a skirt in the rear portion (2b).

23. A method of making a device for ejecting a liquid or pasty product as defined in claim 1, **characterized in that** it includes a step of making at least four distinct parts, a lever (1) incorporating a pawl (4), the pawl (4) and the lever (1) being connected by an elastically deformable zone and forming a monolithic structure made of plastic, a body (2) comprising a front portion (2a) forming a container holder and a rear portion (2b) receiving a rack (3), and **in that** it includes a method of assembling these parts comprising the following steps:
a) inserting the lever onto the rear portion (2b) of the body (2) by inserting its rear cylindrical end (5) in a cylindrical seat (6) of corresponding diameter formed in the rear portion (2b) of said body,
b) inserting the rack (3) into a bore (20) in the rear portion (2b), and
c) connecting the container holder (2a) onto the rear portion (2b) of the body.

24. The method of making an ejection device as claimed in the preceding claim, **characterized in that** it comprises at least three steps of injection molding a plastic to form three separate portions of the device, a lever (1) incorporating a pawl (4), a front portion forming a container holder (2a) and a rear portion (2b) receiving the rack (3).

25. The method of making a device for ejecting a liquid or pasty product as claimed in the preceding claim, **characterized in that** it comprises a fourth step of injection molding a plastic to form said rack (3).
